# EUROPEAN PATENT APPLICATION

(11) **EP 1 736 781 A1**
(43) Date of publication of application: **27.12.2006**
(21) Application number: 05730613.6
(22) Date of filing: 14.04.2005
(51) Int. Cl.: G01N 33/68, C12Q 1/37, G01N 33/533, G01N 33/58

(54) **EFFECTIVE METHOD OF FUNCTION ANALYSIS AND SCREENING OF PROTEIN UTILIZING FLUORESCENT GENERATED BY CELL-FREE PROTEIN SYNTHESIZING SYSTEM**

(30) Priority: 16.04.2004 JP 2004121886
(71) Applicant: Olympus Corporation, Tokyo 151-0072 (JP); CellFree Sciences Co., Ltd., Yokohama-shi, Kanagawa 230-0046 (JP)
(72) Inventor: KOBAYASHI, Tamiyo,c/o Int. Prop. Dep. Olympus Cor, Tokyo 192-8512 (JP); ENDO, Yaeta,c/o CellFree Sciences Co. Ltd, Yokohama, Kanagawa 230-0046 (JP); SAWASAKI, Tatsuya,c/o CellFree Sciences Co Ltd, Yokohama, Kanagawa 230-0046 (JP)
(74) Representative: von Hellfeld, Axel
(86) International application number: PCT/JP2005/007256
(87) International publication number: WO 2005/101015

(57) **Abstract**

A method of detecting a reaction between a fluorescently labeled protein synthesized in a cell-free protein synthesizing system and a sample solution simply, in a short time and at high precision is provided. A case of detecting a binding reaction between an antibody fused with GFP and a sugar on the nanoparticle surface is explained. In a well of a microplate 3, a solution containing an antibody 1 fused with GFP, and a solution containing a nanoparticle 2 with a sugar reactive with the antibody fused with GFP adhered to a surface thereof are mixed to prepare a mixed solution A, and after the reaction, FCS measurement is performed.

## Description

### Technical Field

The present invention relates to a method of analyzing a function of a protein artificially synthesized by utilizing nucleic acid, particularly, in a wheat germ under cell-free, and more specifically, to a method of performing analysis using FCS or FIDA.

### Background Art

As a method of analyzing a function of an artificially expressed protein, there are a method of separating a protein by a gel electrophoresis method after the reaction, and detecting presence or absence of a reaction from mobility of the protein to determine the function, and a method of labeling a subject reactant with a radioisotope, followed by autoradiographic detection.

Patent Document 1 (Jpn. Pat. Appln. KOKAI Publication No. 2000-236896) describes a cell-free protein synthesizing system using a wheat germ extract.

Patent Document 2 (WO 01/016600) describes binding of a fluorescent substance to a C-terminal of a synthesized protein part via an acceptor part by the presence of a labeling reagent at a suitable concentration, in a cell-free translation system using a wheat germ extract or the like. An example of a labeling substance includes a protein, and an example of a method of analyzing interaction between a protein and a molecule includes fluorescence correlation spectroscopy.

However, Patent Documents 1 and 2 do not describe that a sample solution contains beads in a method of detecting presence or absence of a reaction between a fluorescently labeled protein synthesized by a cell-free protein synthesizing system, and a sample solution.

### Disclosure of Invention

When a function of a protein is analyzed by the prior art, a method utilizing a gel electrophoresis is troublesome in operation and time-consuming. In addition, the number of samples which can be run at once is limited in electrophoresis, and this is not suitable for high-throughput screening.

An object of the present invention is to provide a method of detecting a reaction between a fluorescently labeled protein synthesized by a cell-free protein synthesizing system and a sample solution simply, in a short time and at a better precision.

A feature of the present invention attaining the aforementioned object is in that: a fluorescently labeled protein is synthesized by using a cell-free protein synthesizing system; a solution containing the fluorescently labeled protein synthesized is mixed with a sample solution containing beads having, on a surface thereof, a plurality of reactive groups reactive with the fluorescently labeled protein; and a size, a fluorescence intensity or the number of a substance(s) having a fluorescent label in the mixed solution is obtained by fluorescence correlation spectroscopy or fluorescence intensity distribution analysis.

When the fluorescently labeled protein is binding-reacted with the plurality of reactive groups on the surface of beads, the size of whole molecules containing beads is increased and the fluorescence intensity of the whole molecules containing beads is increased in proportion to the number of fluorescently labeled proteins bound to the reactive groups on the surface of beads. As a consequence, a size, a fluorescence intensity or the number of molecules emitting fluorescent light in a reaction solution is obtained by fluorescence correlation spectroscopy or fluorescence intensity distribution analysis, and from the value, a binding reaction between the fluorescently labeled protein and the reaction group on the surface of beads can be known.

Therefore, according to the feature of the present invention, a protein can be fluorescently labeled without treatment such as chemical modification of a protein, and a reaction test can be performed by utilizing a protein in a solution without performing troublesome operations such as utilization of a radioactive isotope element, an electrophoresis, work of immobilizing molecules on a solid substrate, washing, and purification work. And the reaction test can be performed while a function inherent to a protein is maintained. In addition, a size, a fluorescence intensity or the number of molecules emitting fluorescent light in a reaction solution is obtained by fluorescence correlation spectroscopy or fluorescence intensity distribution analysis. And then, comparing the value thus obtained, possible binding reaction between the fluorescently labeled protein and the reactive group on the surface of beads can be detected. Accordingly, the presence or absence of a reaction between a protein and a sample solution can be detected simply, in a short time and at a better precision.

Many reactive groups can be adhered to the surface of beads. Therefore, when a fluorescently labeled protein and a reaction group are bound, many fluorescently labeled proteins gather around beads, and values of FCS measurement and FIDA measurement are obtained at a better precision. Particularly, assume that, by using a nanoparticle having a specific gravity of around 1.0 and a diameter of 0.1 to 0.5 micrometer as beads, low-molecular molecules having a reactive group are adhered to the nanoparticle surface, and a fluorescently labeled protein synthesized by fusing a relatively large fluorescent protein such as a green fluorescent protein (GFP) is binding-reacted with the low-molecular molecules on the nanoparticle surface. In this case, there is a great difference between a fluorescence intensity of a fluorescently labeled protein before the reaction, and a fluorescence intensity of whole nanoparticles after the reaction, and the presence or absence of a binding reaction can be known easily.

Since fluorescence correlation spectroscopy and fluorescence intensity distribution analysis can performed at a small amount of both of a fluorescently labeled protein and a sample solution as compared with other methods, the presence or absence of a reaction can be known at low cost. As a cell-free protein synthesizing system, a wheat germ cell-free protein synthesizing system may be used, in which a protein is synthesized in a wheat germ extract.

In addition, molecules generated by a reaction are adhered to the surface of beads. Therefore, the whole molecules containing beads are separated from a mixed solution, whereby the molecules generated by the reaction can be recovered from the mixed solution after measurement. Thereupon, beads may be separated by centrifugation.

Alternatively, utilizing magnetic beads, beads may be separated with a magnetic force. Thereupon, without removing unreacted components, an active fluorescently labeled protein can be easily recovered from a crude sample solution (such as an cell extract).

Another feature of the present invention is in that: a solution containing a protein and a sample solution are mixed in a well of a microplate; and regarding the mixed solution in the well, a size, a fluorescence intensity or the number of a substance(s) having a fluorescent label in the mixed solution is obtained by fluorescence correlation spectroscopy or fluorescence intensity distribution analysis.

According to this feature, a size, a fluorescence intensity or the number of the substance(s) having a fluorescent label can be obtained simply in a short time and at a better precision, with respect to the mixed solution in the well on the microplate. From these values, the presence or absence of a reaction between the protein and the sample solution can be detected. For this reason, a reaction test with many kinds of samples is performed at once, the presence or absence of a reaction between the protein and the sample solution can be detected simply and in a short time using fluorescence correlation spectroscopy or fluorescence intensity distribution analysis, without purifying a reaction solution and using the reaction solution as it is. This is effective for performing a reaction test on many specimens in a short time.

Still another feature of the present invention is in that: a protein containing a fluorescent protein is synthesized as a fluorescently labeled protein; and a protein which has been fused with a fluorescent substance other than a fluorescent protein during synthesis is synthesized.

According to this feature, a protein to be expressed in a cell-free protein synthesizing system can be fluorescently labeled without losing their original function, which makes it possible to perform a reaction test between the resulting fluorescently labeled protein and a sample at a better precision. When a green fluorescent protein (GFP) or the like is used as a fluorescent protein, a fluorescently labeled protein can be obtained simply since the GFP is easily fused with a protein to be expressed. In addition, a method of synthesizing a protein fused with GFP by utilizing a cell-free protein synthesizing system can reduce the cost as compared with other methods.

Still another feature of the present invention is in that, upon preparation of a mixed solution, the solution containing particular substance for changing the structure of a protein is mixed. According to this feature, a structure of a protein part of a fluorescently labeled protein is changed by the substance for changing the structure of a protein. Therefore, a binding reaction between the fluorescently labeled protein and a reactive group on the surface of beads is suppressed as compared with the case where the substance is not added. As a consequence, the number of fluorescently labeled proteins gathering around beads is reduced, a molecular weight of molecules emitting fluorescent light containing beads is reduced, and a fluorescence intensity is reduced. Accordingly, from a value of a size, a fluorescence intensity or the number of a substance(s) having a fluorescent label in a reaction solution, the presence or absence and an extent of the effect of suppressing a binding reaction between the protein part and the reactive group by the substance can be known. Alternatively, measurement may be performed in such a manner that: after the reaction, beads are recovered from a mixed solution, and a solution containing the substance is mixed therewith, and after that fluorescence correlation spectroscopy or fluorescence intensity distribution analysis is performed. Examples of the substance for changing a structure of a protein include a reducing agent such as sodium hydrogen cyanide.

Still another feature of the present invention attaining the aforementioned object is in that: a fluorescently labeled protein is synthesized by using a cell-free protein synthesizing system; a solution containing the fluorescently labeled protein synthesized and a sample solution containing a substance for separating a fluorescent protein part and a protein part of the fluorescently labeled protein are mixed; and a size, a fluorescence intensity or the number of a substance(s) having a fluorescent label in the mixed solution is obtained by fluorescence correlation spectroscopy or fluorescence intensity distribution analysis.

When the substance for separating a fluorescent protein part and a protein part is used to separate a fluorescent protein part and a protein part of a fluorescently labeled protein, the fluorescent protein part is detached and freely released into surrounding solution as a molecule emitting fluorescent light. Consequently, the fluorescent protein part becomes smaller size than that of the original fluorescently labeled protein, and also fluorescence intensity is reduced. Then, a size, a fluorescence intensity or the number of the molecules emitting fluorescent light in the reaction solution is obtained by fluorescence correlation spectroscopy or fluorescence intensity distribution analysis, and from the value obtained, it is known whether the fluorescently labeled protein has been separated or not.

Therefore, according to the feature of the present invention, a protein can be fluorescently labeled without treatment such as chemical modification of a protein. By utilizing a protein remain solved in a solution, a reaction test can be performed without performing troublesome operations such as utilization of a radioactive isotope element, an electrophoresis, work of immobilizing molecules on a solid substrate, washing and purification work. A reaction test can be performed while a function inherent to a protein is maintained. At the same time, a size, a fluorescence intensity or the number of molecules emitting fluorescent light in a reaction solution is obtained by fluorescence correlation spectroscopy or fluorescence intensity distribution analysis, and from the value obtained, whether a fluorescently labeled protein has been separated or not can be known. As a consequence, the presence or absence of a reaction between a protein and a sample solution can be detected simply, in a short time and at a better precision.

Assume that GFP as a fluorescent label part, and β-glucuronidase (GUS) as a protein part are synthesized by a cell-free protein synthesizing system to obtain GUS fused with GFP as a fluorescently labeled protein, and a protease is used as a substance for separating a fluorescent protein part and a protein part. In this case, particularly, a diffusion time of molecules emitting fluorescent light, that is, GUS fused with GFP before mixing with a protease is about 350 µs, and a fluorescence intensity is about 80 kHz, while a diffusion time of molecules emitting fluorescent light, that is, a GFP part after mixing is about 200 µs, and a fluorescence intensity is about 40 kHz. Therefore, after mixing with a protease, a size, a fluorescence intensity or the number of molecules emitting fluorescent light in a reaction solution is obtained by fluorescence correlation spectroscopy or fluorescence intensity distribution analysis with respect to molecules emitting fluorescent light in a mixed solution. From the value obtained, the effect of protease separation of GUS fused with GFP can be known.

The presence or absence of a reaction between a protein and a sample solution can be detected by either of obtaining of a size of molecules by using fluorescence correlation spectroscopy or obtaining a fluorescence intensity of molecules by using fluorescence intensity distribution analysis. However, the presence or absence of a reaction can be detected at a better precision by using both of them.

In addition, when a fluorescently labeled protein has an intermediate part such as a peptide between a fluorescent protein part and a protein part, a substance for separating a fluorescent protein part and a protein part of a fluorescently labeled protein may be a substance for splitting such an intermediate part.

According to the present invention, a reaction test can be performed without performing troublesome operations such as utilization of a radioactive isotope element, an electrophoresis, work of immobilizing molecules on a solid substrate, washing, and purification work, and a reaction test can be performed while a function inherent to a protein is maintained. Because of this, a size, a fluorescence intensity or the number of a substance(s) having a fluorescent label can be obtained by fluorescence correlation spectroscopy or fluorescence intensity distribution analysis simply and in a short time, and from these values obtained, the presence or absence of a reaction between a protein and a sample solution can be detected. In the present invention, the presence or absence of a reaction can be known at low cost since analysis can be performed at smaller amounts of both a fluorescently labeled protein and a sample solution as compared with other analysis methods.

According to another feature of the present invention, molecules produced by a reaction can be recovered from a mixed solution after measurement. In addition, when magnetic beads are utilized, an active fluorescently labeled protein can be easily recovered and purified without removing unreacted components from a crude sample solution (such as an unpurified cell extract).

Further, utilizing a microplate allows a test of a reaction with many kinds of samples to be performed at once, and a reaction solution is not purified to be used as it is, whereby the presence or absence of a reaction between a protein and a sample solution can be detected simply and in a short time by using fluorescence correlation spectroscopy or fluorescence intensity distribution analysis. This is effective for performing a reaction test on many specimens in a short time.

Since a protein to be expressed in a cell-free protein synthesizing system can be fluorescently labeled without losing their original function, a reaction test between the resulting fluorescently labeled protein and a sample can be performed at a better precision. When GFP or the like is used, a fluorescently labeled protein can be obtained simply. Further, a method of synthesizing a protein with GFP by utilizing a cell-free protein synthesizing system can reduce the cost as compared with other methods.

According to still another feature of the present invention, a binding reaction between a fluorescently labeled protein and a reactive group on the surface of the beads is suppressed as compared with the case where no substance for changing a structure of a protein is added. Therefore, the number of fluorescently labeled proteins gathering around beads is reduced, a molecular weight of molecules emitting fluorescent light containing beads becomes small, and a fluorescence intensity becomes small. Consequently, from a value of a size, a fluorescence intensity or the number of a substance(s) having a fluorescent label in a reaction solution, it is possible to know the presence or absence and an extent of the effect of suppressing a binding reaction between a protein part and a reactive group by means of a substance for changing a structure of a protein.

According to the present invention, a protein can be fluorescently labeled without treatment such as chemical modification of a protein. A reaction test can be performed by utilizing a protein in a solution without performing troublesome operations such as utilization of a radioactive isotope element, an electrophoresis, work of immobilizing molecules on a solid substrate, washing and purification work. A reaction test can be performed while a function inherent to a protein is maintained, and at the same time, it can be known whether a fluorescently labeled protein has been separated or not. Accordingly, the presence or absence of a reaction between a protein and a sample solution can be detected simply, in a short time and at a better precision.

### Brief Description of Drawings

FIG. 1A is a view showing a binding reaction between an antibody fused with GFP and a sugar on the nanoparticle surface.
FIG. 1B is a view showing the binding reaction between an antibody fused with GFP and a sugar on the nanoparticle surface.
FIG. 2A is a view showing another binding reaction between an antibody fused with GFP and a sugar on the nanoparticle surface.
FIG. 2B is a view showing the another binding reaction between an antibody fused with GFP and a sugar on the nanoparticle surface.
FIG. 3 is a graph showing the number of fluorescent molecules and fluorescence intensity with respect to each solution of Example 1.
FIG. 4 is a diagram showing a binding reaction between a single-chain antibody (scFv) fused with GFP and a sugar of Example 1.
FIG. 5 is a diagram showing a change in a protein steric structure of Example 2.
FIG. 6 is a graph showing the number of fluorescent molecules and fluorescence intensity with respect to each solution of Example 2.
FIG. 7 is a diagram showing cleavage of GUS fused with GFP by a SARS protease of Example 3.
FIG. 8 is a graph showing a diffusion time with respect to each solution of Example 3.
FIG. 9 is a graph showing fluorescence intensity with respect to each solution of Example 3.

### Best Mode for Carrying Out the Invention

### 1. FCS measurement and FIDA measurement

Using a single-molecule fluorescence analysis apparatus, a reaction solution in a microplate well is fluorescence-analyzed by fluorescence correlation spectroscopy (FCS) or fluorescence intensity distribution analysis (FIDA). Although the presence or absence of a reaction can be known by either of fluorescence analysis by spectroscopy (FCS measurement) or fluorescence analysis by fluorescence intensity distribution analysis (FIDA measurement), the presence or absence of a reaction can be known at a better precision when both measurements are performed.

### 1.1 FCS measurement

In FCS measurement, fluctuation of fluorescent molecules in a microregion, and based on the resulting information, a diffusion time is obtained. Since a magnitude of the diffusion time indicates a magnitude of a molecular weight, increase or decrease in a molecular weight is known by comparing a diffusion time between before and after the reaction. Increase in a molecular weight indicates a binding reaction between biomolecules, and decrease in a molecular weight indicates a degradation reaction of a biomolecule. Therefore, by detecting increase in a diffusion time of a fluorescently labeled substance before and after the reaction between a fluorescently labeled protein and a sample, a binding reaction between a fluorescently labeled protein and a sample can be detected. When a solution containing molecules for causing a reaction with molecules emitting fluorescent light is mixed into each of two or more solutions containing the same number of molecules emitting fluorescent light, the presence or absence and an extent of a reaction in each mixed solution can be known by comparing a diffusion time between respective mixed solutions.

However, when a molecular weight of biomolecules reacting with a fluorescently labeled protein among biomolecules in a sample is very small compared with a molecular weight of a fluorescently labeled protein, increase in a molecular weight after a binding reaction is small, and therefore, increase in a diffusion time cannot be detected by FCS measurement. In such the case, it is preferable that a large molecule not influencing on a fluorescently labeled protein is pre-bound to biomolecules in a sample. Since when biomolecules and a fluorescently labeled protein are binding-reacted, a molecular weight of molecules emitting fluorescent light produced after a binding reaction is increased by a sum of biomolecules and large molecules, it becomes possible to detect increase in a diffusion time by FCS measurement. As a large molecule to be pre-bound to a biomolecule, beads such as a nanoparticle may be used.

### 1.1.1 Comparison before and after reaction

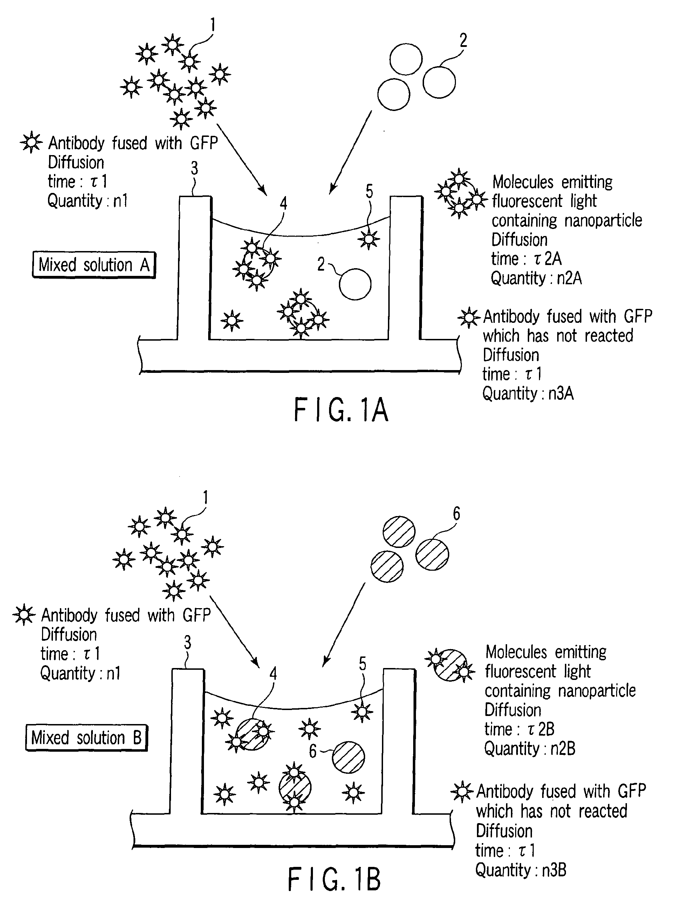

Description will be given to the case where a binding reaction between an antibody fused with GFP and a sugar on the nanoparticle surface is detected before and after the reaction. In a well of a microplate 3, a solution containing antibody 1 fused with GFP and a solution containing nanoparticle 2 with a plurality of immobilized sugars reactive to said antibody 1 on the surface thereof are mixed to prepare a mixed solution A, and FCS measurement is performed after the reaction (see FIGS. 1A and 1B).

When antibody 1 fused with GFP and a plurality of sugars on the surface of nanoparticle 2 are binding-reacted, a plurality of antibodies 1 fused with GFP gather on the surface of one nanoparticle 2. Thus, a diffusion time τ1 and the number n1 of antibodies 1 fused with GFP before the reaction, and a diffusion time τ2A and the number n2A of molecules 4 emitting fluorescent light and containing nanoparticle 2 after the reaction are measured, and the diffusion time τ and the number n are compared between before and after the reaction. This makes it possible to know the presence or absence of a reaction between antibody 1 fused with GFP and the sugar in the mixed solution A.

For example, if τ2A > q1, this measurement result demonstrates that, since a diffusion time of molecule 4 emitting fluorescent light and containing nanoparticle 2 in the mixed solution A after the reaction is great, that is, many antibodies 1 fused with GFP have gathered on the surface of a nanoparticle 2 in the mixed solution A, a molecular weight of the molecule 4 emitting fluorescent light and containing a nanoparticle 2 is increased. Therefore, it is found that an antibody 1 fused with GFP and a sugar on the surface of a nanoparticle 2 have binding-reacted.

In addition, if n1 > n2A > 0, this measurement result demonstrates that, since in the mixed solution A after the reaction, antibodies 1 fused with GFP have gathered on the surface of a nanoparticle 2 to become a molecule 4 as a whole, a total number of molecules emitting fluorescent light is decreased. Therefore, it is found that an antibody 1 fused with GFP and a sugar on the surface of a nanoparticle 2 have binding-reacted.

Also by measuring the number n3 of molecules having no change in diffusion time τ1 between before and after the reaction, that is, antibodies 5 fused with GFP which have not been bound to a nanoparticle after the reaction, and comparing the number before and after the reaction, the presence or absence of a reaction between an antibody 1 fused with GFP and a sugar can be known. For example, if n1 > n3 > 0, this measurement result demonstrates that, in the mixed solution A after the reaction, there are antibodies 5 fused with GFP which have not gathered on the surface of a nanoparticle 2, and the number thereof is smaller than the number n1 of antibodies 1 fused with GFP before the reaction. Therefore, it is found that remaining antibody 1 fused with GFP and a sugar on the surface of a nanoparticle 2 have binding-reacted.

However, if n2A = 0, or n3A = nl, this measurement result demonstrates that antibodies 1 fused with GFP have not gathered on the surface of a nanoparticle 2 in the mixed solution. Consequently, it is found that a binding reaction has not occurred between an antibody 1 fused with GFP and a sugar on the surface of a nanoparticle 2.

Although the presence or absence of a reaction can be known by measuring either of a diffusion time or the number of molecules emitting fluorescent light, the presence or absence of a reaction can be known at a better precision by measuring both of them and comparing them.

### 1.1.2 Comparison between mixed solutions of FCS

Description will be given to the case where a binding reaction between a different kind of sugar and an antibody fused with GFP is compared between two mixed solutions A and B. The mixed solution A is prepared by mixing a solution containing an antibody 1 fused with GFP, and a solution containing a nanoparticle 2 with a sugar reactive with an antibody fused with GFP adhered to a surface thereof. The mixed solution B is prepared by mixing the same solution containing an antibody 1 fused with GFP as that used in the mixed solution A, and a solution containing nanoparticle 6 with a plurality of different sugars from that of the mixed solution A adhered to a surface thereof. Each is prepared in a well of a microplate 3, and FCS measurement is performed to measure a diffusion time τ2 and the number n2 of molecules emitting fluorescent light and containing a nanoparticle after the reaction, and the number n3 of antibodies 5 fused with GFP which have not been reacted. The obtained values are compared between the mixed solutions A and B (see FIGS. 1A and 1B).

For example, if τ2A > τ2B, this measurement result demonstrates that, since a diffusion time of a molecule 4 emitting fluorescent light and containing a nanoparticle 2 in the mixed solution A after the reaction is great, that is, many antibodies 1 fused with GFP have gathered on the surface of a nanoparticle 2 in the mixed solution A than in the mixed solution B, a molecular weight of a molecule 4 emitting fluorescent light and containing a nanoparticle 1 is increased. Therefore, it is found that an antibody 1 fused with GFP and a sugar on the surface of a nanoparticle 2 have binding-reacted better in the mixed solution A than in the mixed solution B.

In addition, if n2A + n3A < n2B + n3B, this measurement result demonstrates that, since a total number of molecules 4 for emitting fluorescent light in the mixed solution A is small, that is, in the mixed solution A, many antibodies 1 fused with GFP have gathered on the surface of a nanoparticle 2 to become a molecule 4 as a whole, a total number of molecules emitting fluorescent light is decreased. Therefore, it is found that an antibody 1 fused with GFP and a sugar on the surface of a nanoparticle 2 have binding-reacted better in the mixed solution A than in the mixed solution B.

By measuring either of a diffusion time or the number of molecules emitting fluorescent light, the presence or absence of a reaction can be known. However, the presence or absence of a reaction can be known at a better precision by measuring both of them and comparing them.

### 1.2 FIDA measurement

When there is a difference in an extent of a binding reaction of an antibody fused with GFP and a sugar on the nanoparticle surface between the mixed solutions A and B, different numbers of antibodies fused with GFP gather on a nanoparticle in the respective mixed solutions A and B. That is, a molecular weight of a nanoparticle on which antibodies fused with GFP have gathered is different between the mixed solutions A and B.

However, a molecular weight of an antibody fused with GFP is much smaller than a molecular weight of a nanoparticle. For this reason, in the case where a difference in molecular weight of a nanoparticle on which antibodies fused with GFP have gathered is small between the mixed solutions A and B, a difference in value is not obtained to such an extent that a difference in molecular weight can be detected, even if a diffusion time is obtained by FCS measurement with respect to molecules emitting fluorescent light present in the mixed solutions A and B. Therefore, it is difficult to detect a difference in an extent of a binding reaction in FCS measurement.

In such a case, an extent of a reaction can be detected by obtaining a fluorescence intensity by means of FIDA measurement. Since a magnitude of the number of antibodies with GFP which have gathered on a nanoparticle changes a magnitude of a fluorescence intensity, a fluorescence intensity of molecules emitting fluorescent light present in the mixed solutions A and B is obtained by means of FIDA measurement, and from the difference in value, a difference in an extent of a binding reaction can be known between the mixed solutions A and B.

Even in the case where a difference in an extent of a binding reaction can be detected by FCS measurement, FIDA measurement is performed jointly to obtain a difference in an extent of a binding reaction from a fluorescence intensity, whereby a difference in an extent of a binding reaction can be known at a better precision.

In FIDA measurement, a fluorescence intensity and the number of molecule emitting fluorescent light in microregion are measured.

When molecules emitting fluorescent light are bound with each other, or when separated into some molecules emitting fluorescent light, a fluorescence intensity emitted by molecules is changed. The number of molecules emitting fluorescent light is also changed. Therefore, by comparing a fluorescence intensity or the number of molecules emitting fluorescent light is compared between before and after the reaction, the presence or absence of a reaction of a molecule can be known. In addition, when a solution containing molecules for causing a reaction with molecules emitting fluorescent light is mixed in each of two or more solutions containing the same number of molecules emitting fluorescent light, the presence or absence and an extent of reaction in each mixed solution can be known by comparing a fluorescence intensity or the number of molecules emitting fluorescent light between the respective mixed solutions.

### 1.2.1 Comparison between before and after reaction of FIDA

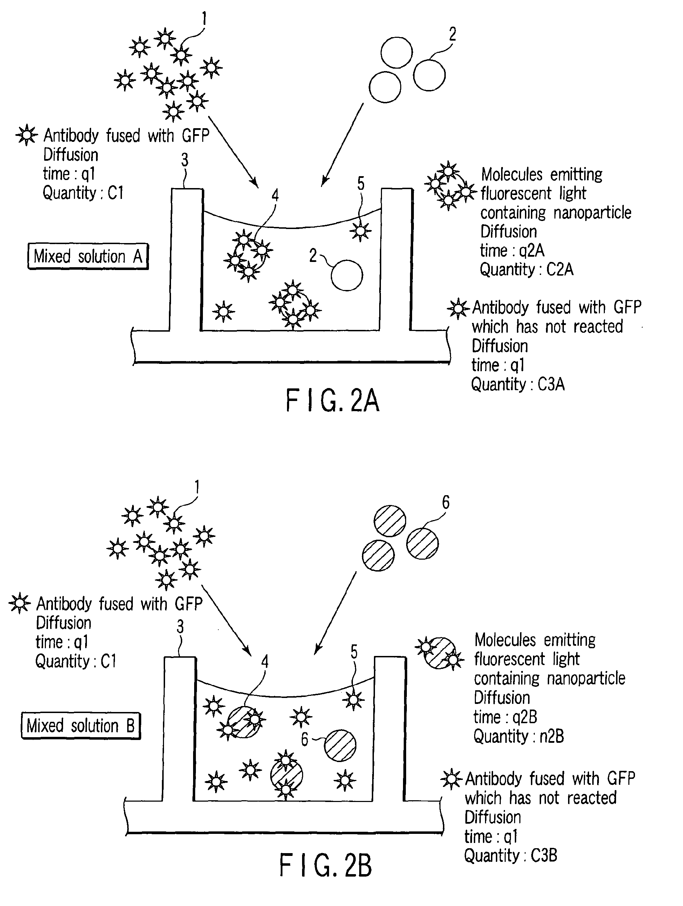

Description will be given to the case where a binding reaction between an antibody fused with GFP and a sugar on the nanoparticle surface is detected before and after the reaction. In a well of a microplate 3, a solution containing an antibody 1 fused with GFP, and a solution containing a nanoparticle 2 with a sugar reactive with the antibody 1 fused with GFP adhered to a surface thereof are mixed to prepare a mixed solution A, and FIDA measurement is performed after the reaction (see FIGS. 2A and 2B).

When an antibody 1 fused with GFP and a sugar on the surface of a nanoparticle 2 are binding-reacted, a plurality of antibodies 1 fused with GFP gather on the surface of one nanoparticle 2. Thus, a fluorescence intensity q1 and the number C1 of an antibody 1 fused with GFP before the reaction, and a fluorescence intensity q2A and the number C2A of molecules 4 emitting fluorescent light and containing a nanoparticle 2 after the reaction are measured, and the fluorescence intensity q and the number C are compared between before and after the reaction, whereby the presence or absence of a reaction between an antibody 1 fused with GFP and a sugar in the mixed solution A can be known.

For example, if q2A > q1, this measurement result demonstrates that, since a fluorescence intensity of a molecule 4 emitting fluorescent light and containing a nanoparticle 2 in the mixed solution A after the reaction is great, that is, many antibodies 1 fused with GFP have gathered on the surface of a nanoparticle 2 in the mixed solution A, a fluorescence intensity of the molecule 4 emitting fluorescent light and containing a nanoparticle 1 has been increased. Therefore, it is found that an antibody 1 fused with GFP and a sugar on the surface of a nanoparticle 2 have binding-reacted.

In addition, if C1 > C2A > 0, this measurement result demonstrates that, since in the mixed solution A after the reaction, antibodies 1 fused with GFP have gathered on the surface of a nanoparticle 2 to become a molecule 4 as a whole, a total number of molecules emitting fluorescent light has been decreased. Therefore, it is found that an antibody 1 fused with GFP and a sugar on the surface of a nanoparticle 2 have binding-reacted.

Also by measuring the number C3 of molecules having no change in fluorescence intensity q1 from before the reaction, that is, antibodies 5 fused with GFP which have not been bound to a nanoparticle after the reaction, and comparing the number between before and after the reaction, the presence or absence of a reaction between an antibody 1 fused with GFP and a sugar can be known. For example, if C1 > C3 > 0, this measurement result demonstrates that, in the mixed solution A after the reaction, there are antibodies 5 fused with GFP which have not gathered on the surface of a nanoparticle 2, and the number is smaller than the number C1 of antibodies 1 fused with GFP before the reaction. Therefore, it is found that remaining antibody 1 fused with GFP and a sugar on the surface of a nanoparticle 2 have binding-reacted.

However, if C2A = 0, or C3 = Cl, this measurement result demonstrates that antibodies 1 fused with GFP have not gathered on the surface of a nanoparticle 2 in the mixed solution. Therefore, it is found that a binding reaction has not occurred between an antibody 1 fused with GFP and a sugar on the surface of a nanoparticle 2.

By measuring either of a fluorescence intensity or the number of molecules emitting fluorescent light, the presence or absence of a reaction can be known. However, the presence or absence of a reaction can be known at a better precision by measuring both of them and comparing them.

### 1.2.2 Comparison between mixed solutions of FIDA

Description will be given to the case where a binding reaction between a different kind of sugar and an antibody fused with GFP is compared between two mixed solutions A and B. The mixed solution A is prepared by mixing a solution containing an antibody 1 fused with GFP, and a solution containing a nanoparticle 2 with a sugar reactive with the antibody 1 fused with GFP adhered to a surface thereof. The mixed solution B is prepared by mixing a solution containing the same antibody 1 fused with GFP as that used in the mixed solution A, and a solution containing a nanoparticle 6 with a different sugar from that of the mixed solution A adhered to a surface thereof. Each is prepared in a well of a microplate 3, and FIDA measurement is performed to measure a fluorescence intensity q2 and the number C2 of molecules emitting fluorescent light and containing a nanoparticle after the reaction, and the number C3 of antibodies 5 fused with GFP which have not been reacted. The obtained values are compared between the mixed solutions A and B (see FIGS. 2A and 2B).

For example, if q2A > q2B, this measurement result demonstrates that a fluorescence intensity of a molecule 4 emitting fluorescent light in the mixed solution A is great, that is, many antibodies 1 fused with GFP have gathered on the surface of a nanoparticle 2 in the mixed solution A. Therefore, it is found that an antibody 1 fused with GFP and a sugar on the surface of a nanoparticle 2 have binding-reacted better in the mixed solution A than in the mixed solution B.

Further, if C2A + C3A < C2B + C3B, this measurement result demonstrates that, since a total number of molecules 4 emitting fluorescent light in the mixed solution A is small, that is, in the mixed solution A, many antibodies 1 fused with GFP have gathered on the nanoparticle surface to become a molecule 4 as a whole, and a total number of molecules emitting fluorescent light is decreased. Therefore, it is found that an antibody 1 fused with GFP and a sugar on the surface of a nanoparticle 2 have binding-reacted better in the mixed solution A than in the mixed solution B.

By measuring either of a fluorescence intensity or the number of molecules emitting fluorescent light, the presence or absence of a reaction can be known. However, the presence or absence of a reaction can be known at a better precision by comparing both of them and comparing them.

As described above, the presence or absence of a binding reaction can be known between before and after the reaction by means of FCS measurement or FIDA measurement, and the presence or absence and an extent of a reaction can be known between mixed solutions.

### Examples

### (Example 1) Interaction between sugar and single-chain antibody

In the present Example, interaction between a sugar and a single-chain antibody (scFv) synthesized in a cell-free protein synthesizing system is detected by FCS measurement and FIDA measurement.

### (1) Preparation of single-chain antibody fused with GFP

Using a wheat germ cell-free protein synthesizing system as a cell-free protein synthesizing system, a protein in which a green fluorescent protein (GFP) is fused with a single-chain antibody (scFv) of an anti-Salmonella antibody was synthesized in a wheat germ extract.

### (2) Preparation of sugar

As a sugar, sugar chains of a Salmonella antigen, a galactose antibody, an Escherichia coli antigen and a mannose antigen are used. These were adhered to surfaces of different nanoparticles (Bangs beads: amino group-modified microsphere PA03N, particle diameter of 500 nm), respectively, to prepare nanoparticles with a sugar.

### (3) Reaction between single-chain antibody fused with GFP and sugar

A solution of a single-chain antibody (scFv) fused with GFP (concentration: 200 nM) and a solution of a nanoparticle with a sugar (concentration: 200 *µ*M) are mixed in a well of a microplate to react them at a room temperature for 15 minutes, to prepare 15 *µ*L of a reaction solution. After the reaction, 24 µL of 50 mM Tris-HCl (pH 8.0) is added (total amount: 39 µL), and FIDA measurement is performed. For comparison, FIDA measurement of a solution in which a nanoparticle with no sugar adhered thereto is mixed, is also performed.

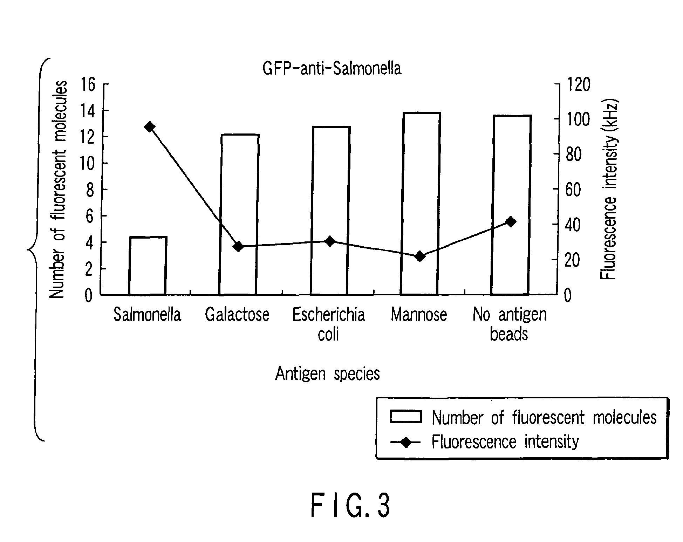

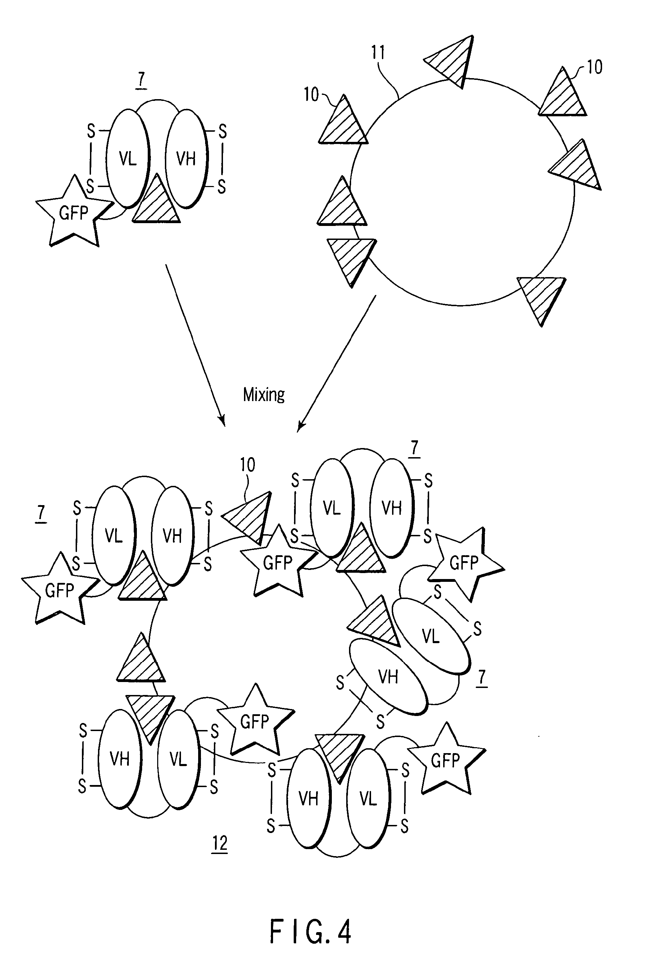

Regarding each solution, a fluorescent molecule number and a fluorescence intensity of molecules emitting fluorescent light, contained in each solution, are shown in FIG. 3. In a reaction with a nanoparticle 11 with a sugar chain 10 of a Salmonella antigen adhered thereto, a fluorescence intensity was increased, and further, a fluorescent molecule number was decreased. Increase in the fluorescence intensity demonstrates that many single-chain antibodies 7 fused with GFP have gathered on the surface of the nanoparticle 11 (see FIG. 4). In addition, decrease in the fluorescent molecule number demonstrates that, since single-chain antibodies 7 fused with GFP have gathered on the nanoparticle surface to become a molecule 12 as a whole, a total number of molecules emitting fluorescent light has been decreased. Therefore, it is found that a GFP-fused anti-Salmonella antibody and a sugar of a Salmonella antigen on the nanoparticle surface have binding-reacted.

On the other hand, in any reaction with a nanoparticle having a sugar chain of a galactose antigen, an Escherichia coli antigen or a mannose antigen adhered thereto, values of a fluorescence intensity and a fluorescent molecule number of substantially the same extent as that of the case of a mixed solution of a nanoparticle with no sugar adhered thereto were obtained. Therefore, it was seen that other antigen species and a GFP-fused anti-Salmonella antibody have not binding-reacted.

Like the present Example, upon detection of a binding reaction between an antibody fused with GFP and a sugar, a sugar is adhered to a large molecule such as a nanoparticle in advance, a solution of an antibody fused with GFP and a solution of a nanoparticle with a sugar are mixed, and FIDA measurement is performed on a reaction solution. As a result, a size, a fluorescence intensity or the number of substances having a fluorescent label can be obtained simply, in a short time and at a better precision, and from these values, the presence or absence of a reaction between an antibody fused with GFP and a sugar can be detected. Since this method can perform analysis at a small amount of both the solution of an antibody fused with GFP and the sample solution, the presence or absence of a reaction can be known at low cost.

Detection of reaction suppression by reducing agent

Now, description will be given to detection of reaction suppression by a substance for changing a structure of a protein part of an antibody fused with GFP.

In a well of a microplate, a solution containing an antibody fused with GFP, a solution containing a nanoparticle with a sugar reactive with an antibody fused with GFP adhered to a surface thereof, and a solution containing a substance for changing a structure of a protein part of an antibody fused with GFP are mixed to prepare a mixed solution A, and FCS measurement or FIDA measurement is performed after the reaction.

When an antibody fused with GFP and a sugar on the nanoparticle surface have binding-reacted, a plurality of antibodies with GFP gather on the surface of one nanoparticle. However, since a structure of a protein part of a fluorescently labeled protein is changed by a substance for changing a structure of a protein, binding between a sugar and an antibody fused with GFP is suppressed. As a consequence, the number of antibodies with GFP which gather on the surface of a nanoparticle is decreased, that is, a molecular weight of molecules emitting fluorescent light and containing beads becomes small, and a fluorescence intensity becomes small. Therefore, after the reaction, FCS measurement or FIDA measurement is performed on a molecule emitting fluorescent light and containing beads, to obtain a diffusion time, a fluorescence intensity or the number, and the obtained value is compared with a value in the case where no substance for changing a structure of a protein is mixed. Consequently, the presence or absence and an extent of the effect of suppressing a binding reaction between a protein part and a reactive group can be known.

Alternatively, measurement may be performed by fluorescence correlation spectroscopy or fluorescence intensity distribution analysis in such a manner that, after a solution containing an antibody fused with GFP and a solution containing a nanoparticle with a sugar reactive with an antibody fused with GFP adhered to a surface thereof are mixed to react them, beads are recovered from the mixed solution, and a solution containing a substance for changing a structure of a protein are mixed.

### (Example 2) Reduction of s-s bond with DTT

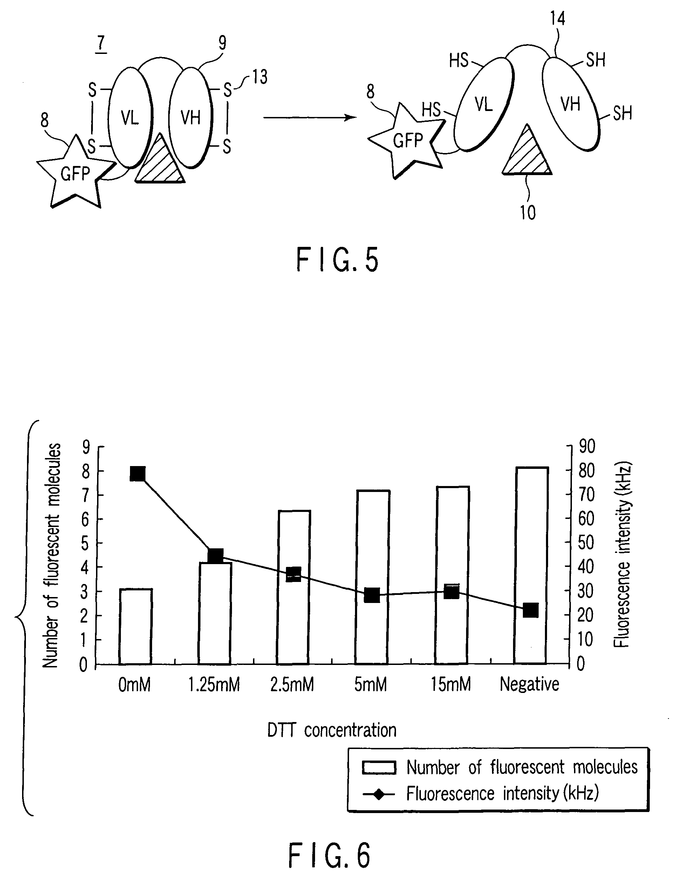

In the present Example, suppression of a binding reaction between a single-chain antibody (scFv) with GFP and a sugar by means of a reducing agent is detected. DTT is used as a reducing agent. A GFP-fused anti-Salmonella antibody is used as a single-chain antibody (scFv) 7 with GFP. As a sugar, a nanoparticle with a sugar chain of a Salmonella antigen adhered to a surface thereof is used. A reducing agent such as DTT (Dithiothreitol) reduces a s-s bond (disulfido bond) of an antibody to change a steric structure of a protein 9. When a steric structure of a protein is changed, activity of the antibody function is lost, and a binding reaction between a protein 14 and a sugar 10 becomes difficult to occur (see FIG. 5).

A solution of a GFP-fused anti-Salmonella antibody, a solution of a nanoparticle with a sugar of a Salmonella antigen and a DTT solution were mixed in a well of a microplate. After the reaction, FIDA measurement was also performed. As a mixed solution, a few kinds of solutions were prepared by varying a concentration of DTT to be added.

FIG. 6 shows a fluorescent molecule number and a fluorescence intensity of molecules emitting fluorescent light with respect to each solution. As the concentration of DTT to be added is greater, a fluorescence intensity is smaller, and a fluorescent molecule number is larger.

A small fluorescence intensity indicates that the number of GFP-fused anti-Salmonella antibodies which have gathered on the nanoparticle surface is small. In addition, a great fluorescent molecule number indicates that GFP-fused anti-Salmonella antibodies do not gather on the nanoparticle surface, and are floating in a solution. Therefore, it is found that addition of DTT has suppressed a binding reaction between a GFP-fused anti-Salmonella antibody and a sugar of a Salmonella antigen on the nanoparticle surface, that is, an antigen-antibody binding reaction is weakened.

Therefore, it is preferable that, when it is detected whether or not a protein has a s-s bond, FIDA measurement is performed in such a manner that a solution containing a fluorescently labeled protein prepared in a cell-free system, a solution containing a substance which binding-reacts with the protein, and a solution of a reducing agent which cleaves s-s bond such as DTT are mixed. From a fluorescence intensity and the number of molecules emitting fluorescent light in a mixed solution, an extent of binding suppression with a reducing agent can be detected, and the presence or absence of a s-s bond of a protein can be known. It is also preferable that, when it is detected whether or not a steric structure of a protein influences on interaction with other molecules, FIDA measurement is performed in such a manner that a substance for changing a steric structure of a protein is added to a mixed solution of a solution containing a fluorescently labeled protein prepared in a cell-free system and a solution containing a substance which binding-reacts with the protein.

Further, it is better that, when a protein specifically binding to a particular substance is detected, FIDA measurement is performed in such a manner that a solution of a reducing agent which cleaves a s-s bond such as DTT is mixed into a solution containing a nanoparticle with proteins gathered on a surface thereof.

When a reducing agent is mixed, a steric structure of a protein which was specifically bound to a particular substance on the nanoparticle surface is changed by a reducing agent. For this reason, specific binding with a particular substance is broken, so that the protein is separated from the nanoparticle surface. On the other hand, a protein which has not been specifically bound to a particle substance on the nanoparticle surface, for example, a protein adhered to the nanoparticle surface is not separated from the nanoparticle surface even when its steric structure is changed by a reducing agent. If FIDA measurement is performed before and after mixing with a reducing agent, a fluorescence intensity of a molecule emitting fluorescent light and containing a nanoparticle after mixing becomes small, and a total number of molecules emitting fluorescent light is increased, it results in that a particle was specifically bound to a particular substance on the nanoparticle surface.

Therefore, by mixing a reducing agent into a sample solution in which a substance on the nanoparticle surface and a protein were reacted in advance, a protein specifically bound to a particular substance on the nanoparticle surface can be screened.

### Cleavage of antibody fused with GFP by protease

Then, regarding a substance for separating a fluorescent protein part and a protein part of an antibody fused with GFP, detection of a separation reaction will be explained. In a well of microplate, a solution containing an antibody fused with GFP, and a solution containing a substance for separating a fluorescent protein part and a protein part are mixed to prepare a mixed solution. After the reaction, FCS measurement or FIDA measurement is performed.

When a substance for separating a fluorescent protein part and a protein part has acted on an antibody fused with GFP, an antibody fused with GFP is separated into a fluorescent protein part and a protein part, and consequently, a fluorescent protein part becomes a molecule emitting fluorescent light in a mixed solution. That is, a molecular weight of a molecule emitting fluorescent light becomes small between before and after the reaction. Further, a fluorescence intensity of a molecule emitting fluorescent light is changed between before and after the reaction. Accordingly, after the reaction, FCS measurement or FIDA measurement is performed on a molecule emitting fluorescent light to obtain a diffusion time, a fluorescence intensity or the number, and the obtained value is compared with a value of the case where no substance for separating a fluorescent protein part and a protein part is mixed, whereby a separation reaction can be detected.

### (Example 3) Measurement of activity of protease

In a cell-free wheat germ protein synthesizing system, GUS 16 (β-glucuronidase) fused with GFP 15 which is a GFP-fused SARS protein was synthesized to obtain a solution A containing GUS 16 fused with GFP 15. This solution and a solution containing a SARS protease were mixed in a well of a microplate to obtain a reaction solution B.

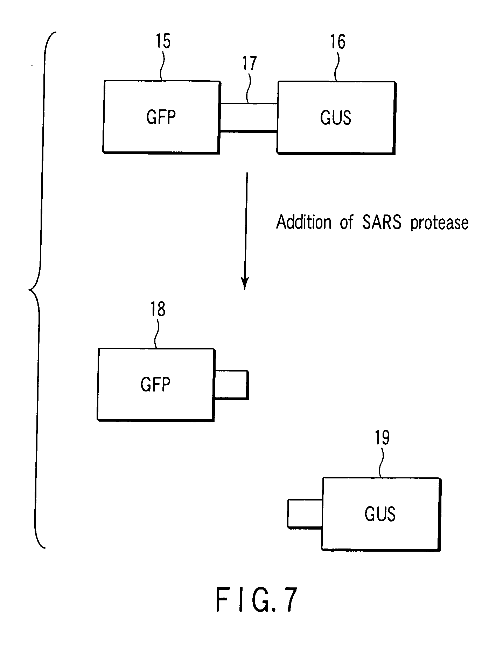

GUS fused with GFP has a SARS protease cleaving site 17 between GFP 15 and GUS 16 regions. A SARS protease has a function of cleaving into a GFP part 18 and a GUS part 19 at the SARS protease cleaving site 17 (see FIG. 7).

FCS measurement and FIDA measurement were performed on the solution A and the reaction solution B. FCS measurement was performed later five times under the condition of irradiation of laser light having a wavelength of 488 nm and an output of 300 *µ*W for 10 seconds per one time. FIDA measurement was performed five times under the condition of irradiation of laser light having a wavelength of 488 nm and an output of 300 *µ*W for 2 seconds per one time.

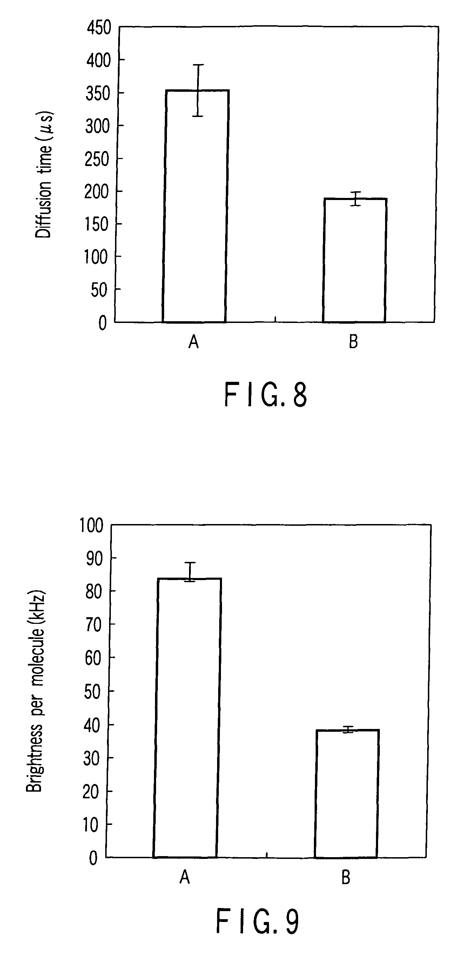

Regarding the solution A (before reaction) and the reaction solution B (after reaction), results of FCS measurement are shown in Table 1, and results of FIDA measurement are shown in Table 2. With respect to the reaction A (before reaction) and the reaction solution B (after reaction), a diffusion time of a molecule emitting fluorescent light, contained in each solution, is shown in FIG. 8, and a fluorescence intensity is shown in FIG. 9.

**Table 1**

| | Translation diffusion time [µs] | SD Difft. K1 | Frac. Triplet [%] | Difft. Triplet [µs] | CR [kHz] | CPP [kHz] | SD Countrate [kHz] | Number of molecules |
|---|---|---|---|---|---|---|---|---|
| GFP only | 75.9 | 1.73 | 1.73 | 55 | 59.2 | 55 | 0.32 | 1.1 |
| Solution A | 354 | 37.99 | 15.7 | 5 | 52 | 38 | 0.9 | 1.4 |
| Solution B | 188.5 | 10.47 | 16.4 | 3.1 | 29.4 | 33.2 | 0.81 | 0.9 |

**Table 2**

| | Fluorescence intensity [kHz] | SD Q1 | C1 | SD C1 | Count rate [kHz] | SD Count rate [kHz] |
|---|---|---|---|---|---|---|
| Solution A | 83.42 | 4.499 | 1.419 | 0.051 | 118.4 | 4.09 |
| Solution B | 38.18 | 0.791 | 1.53 | 0.013 | 61.1 | 2.63 |

A diffusion time of a molecule emitting fluorescent light in the solution A is about 350 *µ*s and a fluorescence intensity is about 80 kHz, while a diffusion time of a molecule emitting fluorescent light in the reaction solution B is about 200 *µ*s and a fluorescence intensity is about 40 kHz.

Since a diffusion time of a molecule emitting fluorescent light is decreased after the reaction, it is found that a molecular weight of a molecule emitting fluorescent light is reduced. Therefore, it is found that a molecule emitting fluorescent light, that is, GUS fused with GFP has been cleaved with a SARS protease. Also since a fluorescence intensity of a molecule emitting fluorescent light is reduced, it is found that GUS fused with GFP has been cleaved with a SARS protease, that is, a SARS protease has the activity.

Consequently, when screening a substance for inhibiting a SARS protease by an inhibiting assay method, FCS measurement or FIDA measurement is performed on a solution obtained by mixing a sample and a SARS protease, whereby a substance for inhibiting a SARS protease can be found out. Thereupon, when both the FCS measurement and FIDA measurement are performed, screening can be performed at a better precision.

### Industrial Applicability

The present invention can be applied to an assay in drug design screening, and has high general utility. Particularly, the present invention is suitable in screening performed while a sample is not treated. An assay can be performed simply, rapidly and at low cost.

## Claims

1. A method of detecting a reaction between a protein and a reactive group, **characterized by** comprising: a step of synthesizing a fluorescently labeled protein by using a cell-free protein synthesizing system; a step of mixing a solution containing the fluorescently labeled protein and a sample solution; and a step of obtaining a size, a fluorescence intensity or the number of a substance(s) having a fluorescent label in the mixed solution by a fluorescence correlation spectroscopy (FCS) or fluorescence intensity distribution analysis (FIDA), wherein the sample solution contains beads having a plurality of reactive groups reactive with the protein on a surface thereof.

2. The method of detecting a reaction between a protein and a reactive group, according to claim 1, **characterized by** further comprising: a step of separating the beads from the mixed solution.

3. The method of detecting a reaction between a protein and a reactive group, according to claim 1, **characterized in that** the mixing step includes a step of mixing the solution containing the protein and the sample solution in a well of a microplate, and a size, a fluorescence intensity or the number of the substance(s) having a fluorescent label is obtained in the mixed solution in the well.

4. The method of detecting a reaction between a protein and a reactive group, according to claim 1, **characterized in that** the step of synthesizing a protein includes a step of synthesizing the protein in a wheat germ extract.

5. The method of detecting a reaction between a protein and a reactive group, according to claim 1, **characterized in that** the fluorescently labeled protein is a protein containing a fluorescent protein, or a protein fused with a fluorescent substance other than a fluorescent protein.

6. The method of detecting a reaction between a protein and a reactive group, according to claim 1, **characterized in that** the mixing step includes a step of mixing a solution containing a substance which changes a structure of the protein.

7. A method of detecting a reaction between a protein and a sample solution, **characterized by** comprising: a step of synthesizing a fluorescently labeled protein by using a cell-free protein synthesizing system; a step of mixing a solution containing the fluorescently labeled protein and a sample solution; and a step of obtaining a size, a fluorescence intensity or the number of a substance(s) having a fluorescent label in the mixed solution by fluorescence correlation spectroscopy (FCS) or fluorescence intensity distribution analysis (FIDA), wherein the sample solution contains a substance which separates a fluorescent protein part and a protein part of the fluorescently labeled protein.
